**Europäisches Patentamt**

**European Patent Office**

**Office européen des brevets**

(19)

(11) Veröffentlichungsnummer: **0 113 084**
**B1**

## EUROPÄISCHE PATENTSCHRIFT

(12)

(45) Veröffentlichungstag der Patentschrift:
**19.02.86**

(21) Anmeldenummer: **83112581.0**

(22) Anmeldetag: **14.12.83**

(51) Int. Cl.⁴: **C 07 C  51/377,** C 07 C  57/04,
B 01 J  27/18, B 01 J  23/72

(54) Verwendung von Mo-V-Cu-P-Katalysatoren zur Oxydehydrierung von Isobuttersäure oder deren Estern.

(30) Priorität: **30.12.82  DE 3248600**

(43) Veröffentlichungstag der Anmeldung:
**11.07.84 Patentblatt 84/28**

(45) Bekanntmachung des Hinweises auf die Patenterteilung:
**19.02.86 Patentblatt 86/8**

(84) Benannte Vertragsstaaten:
**DE FR GB IT NL**

(56) Entgegenhaltungen:
**EP - A - 0 079 491**
**DE - A - 1 204 659**
**DE - A - 2 722 375**
**FR - A - 1 366 300**
**GB - A - 847 784**

(73) Patentinhaber: **Röhm GmbH, Kirschenallee
Postfach 4242, D-6100 Darmstadt 1 (DE)**

(72) Erfinder: **Gruber, Wilhelm, Dr.,
Peter-Behrens-Strasse 34, D-6100 Darmstadt (DE)**

## Beschreibung

Die Erfindung betrifft die Oxydehydrierung von Isobuttersäure oder ihren Estern zu Methacrylsäure oder ihren Estern unter Verwendung von Mo-V-Cu-P-Katalysatoren.

Nach DE-OS Nr. 2722375 werden dafür Katalysatoren verwendet, die durch Eintrocknen einer hydrothermisch erzeugten wässerigen Lösung einer Heteropolysäure der Zusammensetzung $H_5Mo_{10}V_2PO_{40}$ und eines Kupfersalzes, ggf. in Gegenwart eines Alkalisalzes, und Kalzinieren des Rückstandes erhalten worden sind. Das Kupfersalz kann getrennt von der wässerigen Heteropolysäurelösung auf den Träger aufgebracht und in einer eigenen Verfahrensstufe getrocknet werden oder der wässerigen Heteropolysäurelösung nach deren Herstellung zugesetzt werden. Mit diesen Katalysatoren wurden Selektivitäten an Methacrylsäure von 71 bis 72%, bei Mitverwendung von Lithiumsalz sogar von 74,8% erreicht.

Gemäss US Nr. 4307247 werden für die gleiche Dehydrierungsreaktion Katalysatoren aus den Elementen Mo, V, Cu, P, Bi, einem Alkalimetall der Gruppe K, Rb, Cs und ggf. weiteren Metallen sowie Sauerstoff in bestimmten Mengenverhältnissen eingesetzt. Ähnliche Katalysatoren aus den Elementen Mo, V, Cu, P, einem Alkalimetall der Gruppe K, Rb, Cs und einem oder mehreren weiteren Metallen in bestimmten Mengenverhältnissen werden in US Nr. 4314075 für die gleiche Umsetzung vorgeschlagen. Bei der hydrothermischen Umsetzung, die zu einer wässerigen Lösung einer Heteropolysäure führt, sind alle Metallkomponenten anwesend. Die mit diesen Katalysatoren erreichbaren Selektivitäten für Methacrylsäure liegen bei 60 bis 70%.

Auf die gleiche Weise werden Katalysatoren hergestellt, die gemäss US Nr. 4180678 und DE-OS Nr. 3010434 zur Oxidation von Methacrolein zu Methacrylsäure verwendet werden. Sie enthalten vorzugsweise die Elemente Mo, V, Cu, Alkali und P neben Sauerstoff, jedoch sind aus DE-OS Nr. 3010434 auch Katalysatoren ohne Alkalimetall bekannt.

Versuchsweise wurden nach US Nr. 4180678 und DE-OS Nr. 3010434 herstellbare Katalysatoren der Zusammensetzung $Mo_{12}VP_2KCu_{0,3}O_{44,3}$ zur Oxydehydrierung von Isobuttersäure eingesetzt. Bei 290 bis 330° C wurden Selektivitäten von 56 bis 72% erreicht, was keine Verbesserung gegenüber dem Stand der Technik bedeutet.

Obwohl die erzielbaren Ausbeuten an Endprodukt, der Umsatz des eingesetzten Ausgangsprodukts oder die erreichbare Raum-Zeit-Ausbeute wichtige Qualitätsmerkmale eines Katalysators in bezug auf eine bestimmte Umsetzung sind, ist eine hohe Selektivität (d. h. der Quotient aus Ausbeute und Umsatz) das wichtigste Qualitätsmerkmal eines Katalysators, denn sie gibt an, welcher Teil der eingesetzten Ausgangsstoffe tatsächlich in das gesuchte Endprodukt übergeführt wird. Es bestand daher die Aufgabe, die Selektivität bei der Oxydehydrierung von Isobuttersäure zu Methacrylsäure durch Verwendung besserer Katalysatoren zu erhöhen.

Dieses Ziel wird bei der Verwendung eines Katalysators auf Basis von Oxiden des Molybdäns, Vanadins, Kupfers und Phosphors, hergestellt durch hydrothermische Umsetzung von Verbindungen des Molybdäns, Vanadins und Phosphors zu einer wässerigen Lösung einer Heteropolysäure, Eintrocknen der wässerigen Lösung und Kalzinieren des Rückstandes, erfindungsgemäss dadurch erreicht, dass bei der Herstellung des Katalysators die Stufe der hydrothermischen Umsetzung in Abwesenheit von Alkalimetallverbindungen und in Anwesenheit einer Kupferverbindung durchgeführt wird.

Es ist zwar aus der DE-OS Nr. 2722375 bekannt, dass es nicht genügt, die Verbindungen der am Katalysator beteiligten Elemente in Form beliebiger Verbindungen zu kalzinieren, sondern dass zuvor eine Mo-V-Phosphat-Heteropolysäure gebildet werden muss. Da man stets davon ausgegangen ist, dass weitere Metalle, wie z. B. Kupfer oder Kobalt, nicht in die Heteropolysäure eingebaut werden, erschien der Zeitpunkt der Zugabe weiterer Metallverbindungen bei der Herstellung des Katalysators belanglos. Lediglich aus Gründen der Verfahrensvereinfachung sind in manchen Fällen Verbindungen aller beteiligten Elemente gleichzeitig eingesetzt worden, so dass sie auch bei der Bildung der Heteropolysäuren anwesend waren.

Untersuchungen des Erfinders haben gezeigt, dass es für die Aktivität des Katalysators keineswegs gleichgültig ist, welche Metalle neben Mo, V und P bei der hydrothermischen Bildung der Heteropolysäure anwesend sind. Es wurde festgestellt, dass Heteropolysäuren, die in Anwesenheit von Alkalimetallverbindungen gebildet worden sind, beim Kalzinieren weniger aktive Katalysatoren ergeben als in deren Abwesenheit. Für den Kupferanteil gilt das Gegenteil: eine erhöhte Selektivität wird nur erreicht, wenn die Kupferverbindung schon bei der Bildung der Heteropolysäure während der hydrothermischen Umsetzung zugegen ist. Der in dieser Weise hergestellte Kontakt lässt für Methacrylsäure Selektivitäten über 75% und Spitzenwerte bis zu 82% erreichen, was bisher mit keinem anderen Katalysator möglich war.

Der Begriff hydrothermische Umsetzung ist in der DE-OS Nr. 2722375 für die Bildung einer Mo-V-Phosphat-Heteropolysäure aus den Oxiden von Mo, V und P in wässeriger Phase bei 60° C oder mehr während einer meist mehrstündigen Reaktionszeit verwendet worden. Anstelle der Oxide können auch Ammoniummolybdat oder Ammoniumvanadat zur Bildung von Heteropolysäuren verwendet werden. Allerdings haben sich Ammoniumverbindungen für die Katalysatoreigenschaften als ähnlich nachteilig wie Alkaliverbindungen erwiesen, so dass sie vorzugsweise nicht verwendet werden. Die Entstehung der Heteropolysäure verläuft besonders günstig in der Siedehitze unter Normaldruck und ist an einer allmählichen tiefen Rotfärbung der wässerigen Lösung zu erkennen.

Der Begriff Heteropolysäure soll hier nicht auf ganzzahlige stöchiometrische Verhältnisse beschränkt sein. In den reinen Heteropolysäuren liegt ein Mo: P-Verhältnis von 12:1 oder 10:1 vor. Es wurde jedoch festgestellt, dass auch etwas grössere oder kleinere Mo: P-Verhältnisse zu guten Katalysatoren führen. Vermutlich liegen bei P-Unterschuss in der wässerigen Phase neben den eigentlichen Heteropolysäuren auch Mo-Isopolysäuren vor. Das gesamte in Gegenwart von Cu hergestellte Säurengemisch wird hier als Heteropolysäure bezeichnet.

Es ist nich bekannt, welchen Einfluss die Anwesenheit der Cu-Verbindung auf die Bildung der Heteropolysäuren nimmt und ob Cu in die Struktur der Heteropolysäuren eingeschlossen ist. Es genügen ziemlich kleine Mengen an Cu, um den selektivitätssteigernden Effekt herbeizuführen. Bezogen auf Mo liegt der molare Cu-Anteil vorzugsweise bei 0,005 bis 0,1 mol und insbesondere unter 0,05 mol. Die bevorzugten Katalysatoren enthalten die Elemente Mo, V, Cu und P im Verhältnis 12: (0,1-1,5): (0,06-1,2): (0,1-1,5).

Die Cu-haltige Mo-V-P-Heteropolysäurelösung kann als solche in Abwesenheit von Feststoffen eingetrocknet und der Rückstand kalziniert und als Katalysator eingesetzt werden. Häufig wird es vorgezogen, den so erhaltenen Katalysator in einem Gewichtsverhältnis von z. B. 1 zu 0,1 bis 10 mit einem inerten Material, wie feinteiligem $SiO_2$, $Al_2O_3$, $ZrO_2$, Siliciumcarbid u. dgl., zu verschneiden und zu einem körnigen Kontaktmaterial zu verarbeiten, was durch Pressen, Pelletieren oder Granulieren geschehen kann. Das Gemisch kann zu diesem Zweck mit Wasser oder wässerigen Lösungen angeteigt werden. Auch kann man beim Pelletieren von harten, mehr oder weniger unporösen inerten Trägerkörpern, vorzugsweise mit rauher Oberfläche ausgehen und den Katalysator als dünne Schale nach der Dragiertechnik aufbringen. Dazu werden auf die Trägerkörper, z. B. keramische Kügelchen, in einer in Schräglage rotierenden Trommel der pulverförmige Katalysator und eine ggf. bindemittelhaltige Flüssigkeit aufgetragen und die entstehenden Pellets anschliessend getrocknet. Erforderlichenfalls wird der ganze Arbeitsgang mehrfach wiederholt.

Die wässerige Lösung der Heteropolysäure kann auch in Gegenwart eines festen Katalysatorträgermaterials eingetrocknet werden. Dazu eignen sich die oben schon genannten feinteiligen inerten Materialien. Die Trägerstoffe können eine Porosität unter 10 Vol.-% oder/und eine innere Oberfläche über 1 m²/g haben. Der Anteil dieser Träger kann, bezogen auf das Gesamtgewicht des Katalysators, 5 bis 95 Gew.-% ausmachen. Das Trägermaterial wird mit der Heteropolysäurelösung zu einem Brei vermischt und durch Erhitzen auf den Siedepunkt, ggf. bei Unterdruck bei Temperaturen von z. B. 50 bis 110° C zur Trockne eingedampft.

Es genügt, hierbei bis zu einer festen, nach dem Zerkleinern als rieselfähiges Pulver handhabbaren Beschaffenheit zu gelangen, die noch Wasser enthalten kann. In gleicher Weise kann eine von Trägerstoffen freie Lösung der Heteropolysäure eingedampft werden. Das restliche Wasser kann beim Kalzinieren entfernt werden. Dabei wird der Trocknungsrückstand, vorzugsweise in Gegenwart von Sauerstoff während etwa 1 bis 8 h auf 100 bis 350° C erhitzt. Vorzugsweise enthält die wässerige Lösung neben den Elementen Mo, V, Cu, P und O nur noch solche Bestandteile, die sich unter den Kalzinierungsbedingungen verflüchtigen oder in Oxide übergehen; dazu gehören z. B. Chlorid-, Nitrat- oder Acetationen.

Der so erhaltene Katalysator wird in bekannter Weise zur Oxydehydrierung von Isobuttersäure zu Methacrylsäure oder zur Oxydehydrierung der entsprechenden niederen Alkylester, wie Methylisobutyrat, in der Dampfphase eingesetzt. Das Reaktionsgas enthält pro Mol Isobuttersäure bzw. ihrem Ester 1 bis 2 mol Sauerstoff, im allgemeinen in Form von Luft, und 0 bis 3 mol Wasserdampf, und wird bei Temperaturen oberhalb 300° C bis etwa 350° C umgesetzt. Dazu kann ggf. gemäss DE-OS Nr. 3019731 ein Kreislaufreaktor verwendet werden.

I. Herstellung von Katalysatoren

A. Katalysator gemäss der Erfindung. In 1440 g entionisiertem Wasser wurden 144,2 g Molybdäntrioxid (99,8%ig), 9,1 g Vanadiumpentoxid, 1,59 g Kupfer-(II)-oxid und 11,53 g 85%ige Phosphorsäure dispergiert bzw. gelöst. Das so erhaltene Gemisch wurde dann 5 h lang unter Rückfluss und Rühren erhitzt. Dabei wurde eine rotbraune Lösung erhalten, die nach Zugabe von 57,76 g Kieselgur 1 und 11,55 g pyrogener Kieselsäure 2 unter Rühren zur Trockne eingedampft wurde. Die eingeengte Masse wurde 4 h lang bei 200° C getrocknet, dann in ca. 5 mm grosse Granulatstücke zerteilt und diese anschliessend noch 3 h lang bei 300° C erhitzt.

B. Vergleichskatalysator gemäss US Nr. 4180678, Beispiel 1. Zu einer Lösung von 219 g Ammoniumheptamolybdat in 830 g entionisiertem Wasser wurde eine Lösung gegeben, die aus 7,49 g Kupfer-(II)-nitrat in 62 g reinem Wasser und 11,92 g 85%iger Phosphorsäure, verdünnt mit 20 g Wasser hergestellt war. Die vereinigten Lösungen wurden anschliessend unter Rühren 1 h auf 90° C erhitzt, wobei ein hellgrüner Niederschlag ausfiel. Dann wurde eine aus einer Mischung von 11,92 g 85%iger Phosphorsäure, 62 g Wasser, 9,4 g Vanadiumpentoxid durch Eindampfen hergestellte Masse hinzugerührt, das dabei entstandene braune Gemisch noch mit einer Lösung von 5,8 g Kaliumhydroxid in 62 g Wasser versetzt und zum Schluss nach Zugabe von 75 g Kieselgur und 15 g pyrogener Kieselsäure zur Trockne eingeengt. Das feste Produkt
1) Innere Oberfläche 2-10 m²/g
2) Innere Oberfläche 200 m²/g
wurde zunächst 4 h bei 200° C und dann nach Zerteilung in ca. 5 mm grosse Stücke 5 h bei 300° C in Luftatmosphäre erhitzt.

C. Vergleichskatalysator gemäss DE-OS Nr. 3010434, Beispiel 1. Ein Gemisch aus 143,9 g Molybdäntrioxid, 7,59 g Vanadiumpentoxid, 19,22 g 85%ige Phosphorsäure, 6,04 g Kupfer-(II)-nitrat und 8,43 g Kaliumnitrat in 1520 g entionisiertem Wasser wurden 6 h bei 100° C unter Rückfluss erhitzt, wobei eine klare orange-rote Lösung entstand. Diese wurde mit 60,47 g Kieselgur und 12,1 g pyrogener Kieselsäure versetzt, das Gemisch bei ca. 90° C zur Trockne eingeengt, dies anschliessend 4 h bei 200° C und dann nach Zerkleinerung die ca. 5 mm grossen Stücke noch 5 h bei 300° C erhitzt.

II. Oxydehydrierung von Isobuttersäure (IBS)

Es werden Kreislaufreaktoren mit Katalysatorbettdurchmessern von 6-7 cm verwendet. Die Katalysatoren werden in einer Höhe von 2-5 cm eingefüllt. Das eingesetzte Reaktionsgas hat die Zusammensetzung $IBS:O_2:N_2$ im Molverhältnis 1:1,5:20.

| Beispiel Nr. | Katalysator | Katalysatortemperatur (°C) | Durchsatz (Mol IBS/g Katalysator u. Min) | IBS-Umsatz (%) | Selektivität Methacrylsäure (%) |
|---|---|---|---|---|---|
| 1 | A | 310 | $11 \cdot 10^{-5}$ | 62,8 | 76,5 |
| 2 | A | 320 | $14 \cdot 10^{-5}$ | 75,3 | 78,8 |
| 3 | A | 320 | $18 \cdot 10^{-5}$ | 70,7 | 82,5 |
| 4 | A | 330 | $15 \cdot 10^{-5}$ | 79,1 | 82,4 |
| 5 | A | 340 | $18 \cdot 10^{-5}$ | 82,1 | 82,7 |
| 6 | A | 350 | $18 \cdot 10^{-5}$ | 86,1 | 81,5 |
| Vergleichsversuche: | | | | | |
| 7 | B | 290 | $5 \cdot 10^{-5}$ | 77,5 | 72,4 |
| 8 | B | 320 | $12 \cdot 10^{-5}$ | 73,4 | 64,9 |
| 9 | B | 330 | $12 \cdot 10^{-5}$ | 71,4 | 58,5 |
| 10 | C | 320 | $6 \cdot 10^{-5}$ | 70,8 | 70,3 |
| 11 | C | 330 | $11 \cdot 10^{-5}$ | 63,0 | 57,1 |
| 12 | C | 330 | $12 \cdot 10^{-5}$ | 70,9 | 56,6 |

III. Dehydrierungsergebnisse nach DE-OS Nr. 2722375, Beispiel 7, Nrn. 4-6

Gemäss Beispiel 7 wurden mit den Katalysatoren Nrn. 4, 5, 6, die durch Tränken von Diatomeen-Erde mit Cu-Salz- und ggf. Alkalisalzlösungen, Trocknen und Kalzinieren, Tränken mit einer Lösung der Heteropolysäure $H_5Mo_{10}V_2PO_{400}$, Trocknen und Kalzinieren hergestellt worden waren, folgende Selektivitäten für Methacrylsäure aus Isobuttersäure erreicht:

Katalysator 4 (Cu-Nitrat)          71,8%
Katalysator 5 (Cu-Bromid)          71,1%
Katalysator 6 (Cu-Nitrat + $Li_2SO_4$)   74,8%

**Patentansprüche**

1. Verwendung eines Katalysators auf Basis von Oxiden des Molybdäns, Vanadins, Kupfers und Phosphors, hergestellt durch hydrothermische Umsetzung von Verbindungen des Molybdäns, Vanadins und Phosphors zu einer wässerigen Lösung einer Heteropolysäure, Eintrocknen der wässerigen Lösung, ggf. in Gegenwart eines ungelösten Trägerstoffes und Kalzinieren des Rückstandes, zur Oxydehydrierung von Isobuttersäure zu Methacrylsäure oder von niederen Isobuttersäureestern zu den entsprechenden Methacrylsäureestern, dadurch gekennzeichnet, dass ein Katalysator verwendet wird, bei dessen Herstellung die Stufe der hydrothermischen Umsetzung in Abwesenheit von Alkalimetallverbindungen und in Anwesenheit einer Kupferverbindung durchgeführt worden ist.

2. Verwendung eines Katalysators gemäss Anspruch 1, dadurch gekennzeichnet, dass er pro Mol Mo 0,005 bis 0,1 mol insbesondere weniger als 0,05 mol Cu enthält.

3. Verwendung eines Katalysators gemäss den Ansprüchen 1 bis 3, gekennzeichnet durch den Gehalt der Elemente Mo, V, Cu und P im Molverhältnis 12:(0,1-1,5):(0,06-1,2):(0,1-1,5).

4. Verwendung eines Katalysators gemäss den Ansprüchen 1 bis 3, dadurch gekennzeichnet, dass bei seiner Herstellung die wässerige Lösung in Abwesenheit eines Trägerstoffes eingetrocknet worden ist.

5. Verwendung eines Katalysators gemäss den Ansprüchen 1 bis 3, dadurch gekennzeichnet, dass bei seiner Herstellung die wässerige Lösung in Gegenwart eines Trägerstoffes mit einer Porosität unter 10% oder/und einer inneren Oberfläche über 1 $m^2/g$ eingetrocknet worden ist.

6. Verfahren zur Herstellung von Methacrylsäure oder ihren niederen Estern durch Oxydehydrierung von Isobuttersäure oder ihren niederen Estern in der Dampfphase in Gegenwart von Sauerstoff bei einer Temperatur über 300° C in Gegenwart eines Katalysators auf Basis von Oxiden des Molybdäns, Vanadins, Kupfers und Phosphors, hergestellt durch hydrothermische Umsetzung von Verbindungen des Molybdäns, Vanadins und Phosphors zu einer wässerigen Lösung einer Heteropolysäure, Eintrocknen der wässerigen Lösung, ggf. in Gegenwart eines ungelösten Trägerstoffes und Kalzinieren des Rückstandes, dadurch gekennzeichnet, dass ein Katalysator verwendet wird, bei dessen Herstellung die Stufe der hydrothermischen Umsetzung in Abwesenheit von Alkalimetallverbindungen und in Anwesenheit einer Kupferverbindung durchgeführt worden ist.

## Claims

1. Use of a catalyst based on oxides of molybdenum, vanadium, copper and phosphorus, prepared by hydrothermic reaction of compounds of molybdenum, vanadium, and phosphorus to yield an aqueous solution of a heteropolyacid, drying the aqueous solution, optionally in the presence of an undissolved carrier and calcining the residue, for the oxydehydration of isobutyric acid to obtain methacrylic acids or of lower isobutyric acid esters to obtain the corresponding methacrylic acid esters, characterised in that a catalyst is used, the preparation of which has included the step of hydrothermic reaction in the absence of alkali metal compounds and in the presence of a copper compound.

2. Use of a catalyst as claimed in Claim 1, characterised in that per mole of Mo it contains from 0.005 to 0.1 mol, more particularly less than 0.05 mol of Cu.

3. Use of a catalyst as claimed in Claims 1 to 3, characterised in that it contains the elements Mo, V, Cu and P in a molar ratio of 12: (0.1-1.5):(0.06-1.2):(0.1-1.5).

4. Use of a catalyst as claimed in Claims 1 to 3, characterised in that, during its preparation, the aqueous solution has been dried in the absence of a carrier.

5. Use of a catalyst as claimed in Claims 1 to 3, characterised in that, during its preparation, the aqueous solution has been dried in the presence of a carrier having a porosity of less than 10% and/or an internal surface area of more than 1 m²/g.

6. Process for preparing methacrylic acid or the lower esters thereof by oxydehydration of isobutyric acid or the lower esters thereof in the vapour phase in the presence of oxygen at a temperature of above 300° C in the presence of a catalyst based on oxides of molybdenum, vanadium, copper and phosphorus prepared by hydrothermic reaction of compounds of molybdenum, vanadium and phosphorus to obtain an aqueous solution of a heteropolyacid, drying the aqueous solution, optionally in the presence of an undissolved carrier and calcining the residue, characterised in that a catalyst is used in the preparation of which the step of hydrothermic reaction has been carried out in the absence of alkali metal compounds and in the presence of a copper compound.

## Revendications

1. Utilisation d'un catalyseur à base d'oxydes de molybdène, du vanadium, du cuivre et du phosphore, préparé par réaction hydrothermique de composés de molybdène, du vanadium et du phosphore pour l'obtention d'une solution aqueuse d'un hétéropolyacide, séchage de la solution aqueuse, le cas échéant en présence d'une matière de support non dissoute et calcination du résidu, pour l'oxydéshydrogénation d'acide isobutyrique en acide méthacrylique ou d'esters inférieurs d'acide isobutyrique en esters correspondants d'acide méthacrylique, caractérisée en ce qu'on utilise un catalyseur dans la préparation duquel la phase de réaction hydrothermique est effectuée en l'absence de composés de métaux alcalins et en présence d'un composé du cuivre.

2. Utilisation d'un catalyseur selon la revendication 1, caractérisée en ce que celui-ci contient, par mol de Mo, 0,005 à 0,1 mol, en particulier moins de 0,05 mol de Cu.

3. Utilisation d'un catalyseur selon l'une des revendications 1 ou 2, caractérisée par la teneur de celui-ci en les éléments Mo, V, Cu et P dans le rapport molaire 12: (0,1-1,5):(0,06-1,2):(0,1-1,5).

4. Utilisation d'un catalyseur selon l'une des revendications 1 à 3, caractérisée en ce que lors de la préparation de celui-ci, la solution aqueuse a été séchée en l'absence d'une matière de support.

5. Utilisation d'un catalyseur selon l'une des revendications 1 à 3, caractérisée en ce que lors de la préparation de celui-ci, la solution aqueuse a été séchée en présence d'une matière de support ayant une porosité de moins de 10% et/ou une surface interne de plus de 1 m².

6. Procédé pour la préparation d'acide méthacrylique ou de ses esters inférieurs par oxydéshydrogénation d'acide isobutyrique ou de ses esters inférieurs en phase vapeur, en présence d'oxygène, à une température supérieure à 300° C, en présence d'un catalyseur à base d'oxydes du molybdène, du vanadium, du cuivre et du phosphore, préparé par réaction hydrothermique de composés du molybdène, du vanadium et du phosphore pour l'obtention d'une solution aqueuse d'un hétéropolyacide, séchage de la solution aqueuse, le cas échéant en présence d'une matière de support non dissoute, et calcination du résidu, caractérisé en ce qu'il est utilisé un catalyseur dans la préparation duquel la phase de réaction hydrothermique est menée en l'absence de composés de métaux alcalins et en présence d'un composé du cuivre.